# EUROPEAN PATENT SPECIFICATION

(11) **EP 4 312 831 B1**
(45) Date of publication and mention of the grant of the patent: **01.01.2025**
(21) Application number: 22715914.2
(22) Date of filing: 23.03.2022
(51) Int. Cl.: A61B 17/34, A61B 90/30

(54) **ILLUMINATED SURGICAL CANNULA**
BELEUCHTETE CHIRURGISCHE KANÜLE
CANULE CHIRURGICALE ÉCLAIRÉE

(30) Priority: 25.03.2021 US 202163166010 P
(43) Date of publication of application: 07.02.2024
(73) Proprietor: Lumitex, Inc., Strongsville, Ohio 44136 (US)
(72) Inventor: DOMBROWSKI, Joseph, Medina, Ohio 44256 (US); TOICH, Sara, Strongsville, Ohio 44136 (US); DIEHL, Nicolette, Brunswick, Ohio 44136 (US)
(74) Representative: Aldridge, Christopher Simon
(86) International application number: PCT/US2022/021479
(87) International publication number: WO 2022/204240

(56) References cited:
- WO-A1-2015/179708
- US-A1- 2009 036 744

## Description

### Technical Field

The present disclosure relates generally to surgical cannula and more particularly to an illuminated surgical cannula.

### Background

When performing surgery, there is often a tradeoff between tissue damage and accessing and visualizing a surgical location. For example, to more fully view tissues in a surgical location, surgeons may need to make larger incisions to create a larger surgical cavity, as well as use surgical retractors for holding open the surgical cavity.

WO2015/179708 (Invuity Inc) discloses various surgical devices that have integrated means of illuminating a surgical field. Retractors, cannulas, suction devices and the like are disclosed as having integrated optical waveguides that are coupleable to external lighting sources. The optical waveguides feature cladding layers configured to enhance transmission efficiency.

US2009/036744 (Vayser) discloses an illumination system that comprises an arthroscope, endoscope or other suitable surgical tool and an attachable cannula comprising a transparent or semi-transparent material capable of carrying light from the proximal end of the cannula to the distal end of the cannula, via a waveguide, thereby illuminating a surgical field. The surgical field is thus illuminated through components that do not occupy space that may otherwise by used for the optics of the arthroscope.

### Summary

The present disclosure provides a surgical cannula for illuminating a surgical cavity, as set out in accompanying claim 1. Preferred features are set out in the accompanying sub-claim.

Thu, the present invention uses an optically transparent walled passageway and a light source for illuminating the working channel of the walled passageway, such that tissues accessible via the working channel and tissues surrounding the walled passageway are illuminated.

While several features are described herein with respect to embodiments of the invention; features described with respect to a given embodiment also may be employed in connection with other embodiments. The following description and the annexed drawings set forth certain illustrative embodiments of the invention. These embodiments are indicative, however, of but a few of the various ways in which the principles of the invention may be employed. Other objects, advantages, and novel features according to aspects of the invention will become apparent from the following detailed description when considered in conjunction with the drawings.

### Brief Description of the Drawings

The annexed drawings, which are not necessarily to scale, show various aspects of the invention in which similar reference numerals are used to indicate the same or similar parts in the various views.
FIG. 1 is a perspective view of an exemplary embodiment of a surgical cannula including a separate dilator and retainer.
FIG. 2 is a perspective view of the surgical cannula of FIG. 2 with the dilator inserted into the retainer.
FIG. 3A is a perspective view of an exemplary embodiment of the surgical cannula including a light source having multiple light emitters in accordance with the invention.
FIG. 3B is a cut away side view of the surgical cannula of FIG. 3A.
FIG. 4A is a side cut away view of an exemplary embodiment of the surgical cannula including a light source having multiple light emitters.
FIG. 4B is a top view of the surgical cannula of FIG. 4A.
FIG. 5 is a side cut away view of an exemplary embodiment of the surgical cannula illuminated by an external light source.
FIG. 6A is a side cut away view of an exemplary embodiment of the surgical cannula including a light source and a linear illuminator.
FIG. 6B is a top view of the surgical cannula of FIG. 6A.
FIG. 7 is a side view of an exemplary embodiment of the surgical cannula including a light source and a linear illuminator located on an outer surface of the retainer.
FIG. 8A is a side cut away view of an exemplary embodiment of the surgical cannula including a light source and a planar illuminator.
FIG. 8B is a side perspective view of the surgical cannula of FIG. 8A.

The present invention is described below in detail with reference to the drawings. In the drawings, each element with a reference number is similar to other elements with the same reference number independent of any letter designation following the reference number. In the text, a reference number with a specific letter designation following the reference number refers to the specific element with the number and letter designation and a reference number without a specific letter designation refers to all elements with the same reference number independent of any letter designation following the reference number in the drawings.

### Detailed Description

In a general embodiment, the present disclosure provides a surgical cannula for illuminating a surgical cavity using an optically transparent walled passageway having a working channel. Light from a light source is received by the surgical cannula and is emitted by the walled passageway to illuminate the working channel, such that tissues accessible via the working channel and tissues surrounding the walled passageway are both illuminated.

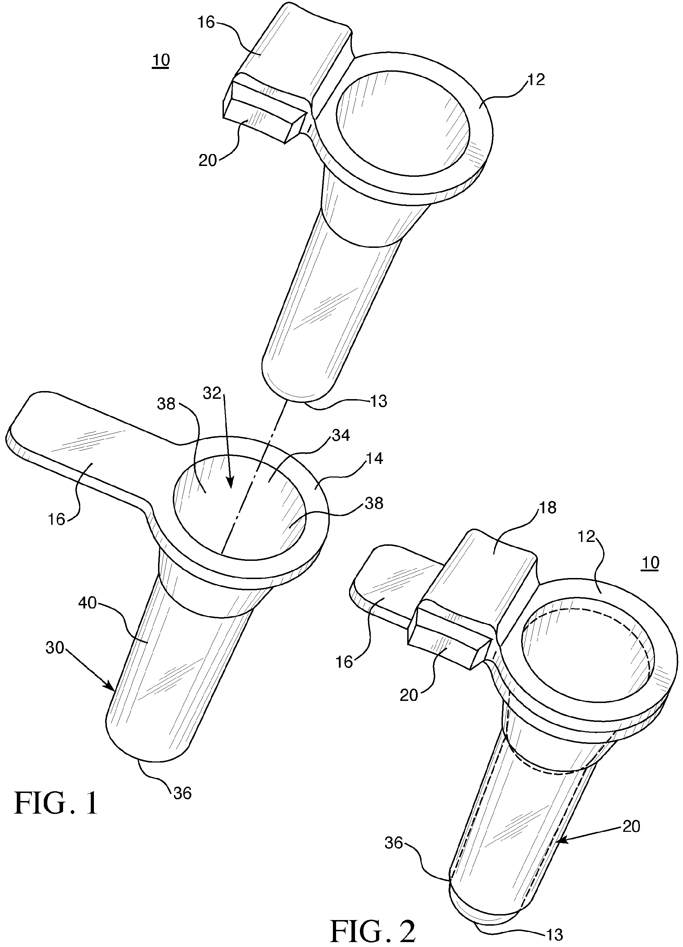

An exemplary embodiment of the surgical cannula 10 (also referred to as a surgical retractor) is shown in FIGS. 1 and 2. In this embodiment, the surgical cannula 10 includes a dilator 12 having a closed tip 13 and a retainer 14 having an open tip 15. In this embodiment, the dilator 12 pushes soft tissue of the brain out of the way as the retainer 14 is advanced into the brain. That is, the dilator 12 may be received within an interior working channel of the retainer 14, such that the closed tip 13 of the dilator 12 extends through and past the open tip 15 of the walled passageway 30 as shown in FIG. 2.

The dilator 12 and retainer 14 may be releasably attached via a clip 16. For example, the clip 16 may attach to a tab 18 of the retainer 14. The dilator 12 may then be removed while the retainer 14 remains in place to retain tissue out of the way and provide a working area for a surgeon. For example, a button 20 of the clip 16 may be depressed to disengage the clip 16 from the tab 18 so that the dilator 14 may be removed to open the working channel 32.

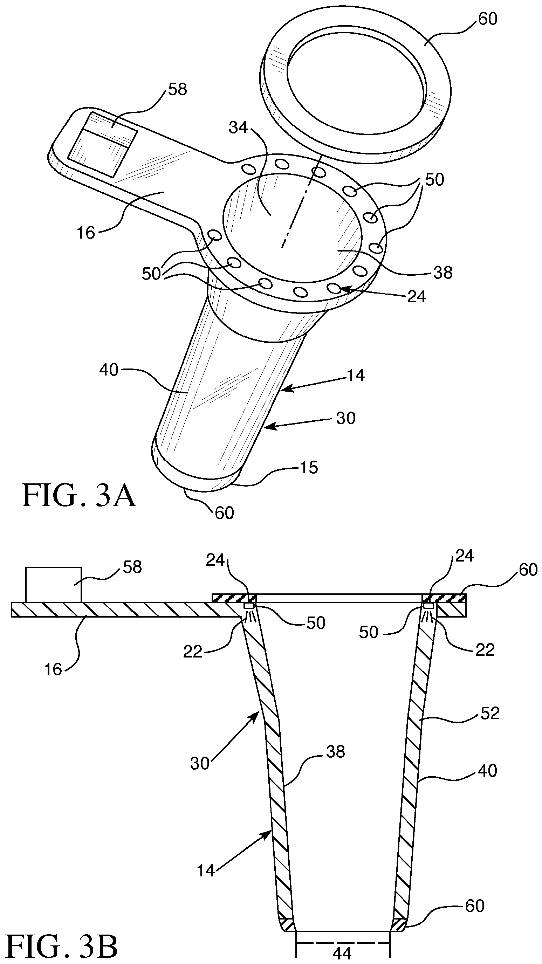

Turning to FIGS. 3A and 3B, an exemplary embodiment is shown of the surgical cannula 10 for illuminating a surgical cavity using light 22 emitted by a light source 24. The surgical cannula 10 includes an optically transparent walled passageway 30 having an interior working channel 32, an entrance 34, an open tip 15, an inner surface 38, an outer surface 40, and light-extracting features 42. The walled passageway 30 receives the light 22 emitted by the light source 24 and transports the received light 22 via total internal reflection towards the open tip 15 of the walled passageway 30. The light-extracting features 42 extract the transported light 22 from the walled passageway 30, such that the extracted light is emitted from the inner surface 38 of the walled passageway 30 into the working channel 32 and illuminates an area 44 bounded by the open tip 15 the walled passageway 30 (also referred to as a working area).

The light-extracting features may include both inward extracting structures and outward extracting structures. The inward extracting structures extract the transported light, such that the extracted light illuminates the area 44 bounded by the open tip 15 of the walled passageway 30. The outward extracting structures extract the transported light, such that the extracted light is emitted from the external surface 40 of the walled passageway 30. In this way, the outward extracting structures may be used to illuminate the environment (e.g., tissues) surrounding the walled passageway 30.

The light source 24 may include multiple light emitters 50 configured to emit the light 22. For example, as shown in FIG. 3A, each of the light emitters 50 may be positioned along the entrance 34 of the walled passageway 30, such that the light 22 emitted by each of the light emitters 50 is transported towards the open tip 15 of the walled passageway 30.

As described above, the walled passageway 30 is optically transparent. That is, the walls 40 of the walled passageway 30 allow at least a portion of the visible spectrum of light to pass through. For example, the walls 40 may be predominantly (e.g., at least 50%) clear. In one example, the walls 40 attenuate at most 10%, at most 20%, or at most 30% of the visible spectrum of light. The walled passageway 30 may be made of any suitable optically transparent material such as glass and/or plastic. In one embodiment, the walled passageway 30 is configured to be sterilized before use.

Each of the light emitters 50 may be enclosed within walls 52 of the walled passageway 30. For example, the light emitters 50 may be positioned within recesses in the walls 52 and the light emitters 50 may then be encapsulated within the recesses.

In one embodiment, the surgical cannula 10 includes a tab 18 integrally formed with the walled passageway 30. The tab 18 may be optically connected to the walled passageway 30 and the light source 24, such that the light 22 emitted by the light source 24 is received by the tab 18 and is transferred by total internal reflection from the tab 18 into the walled passageway 30.
the tab 18 may be any suitable structure mechanically attached to the walled passageway 30. For example, the tab 18 may be integrally or monolithically formed without the walled passageway 30.

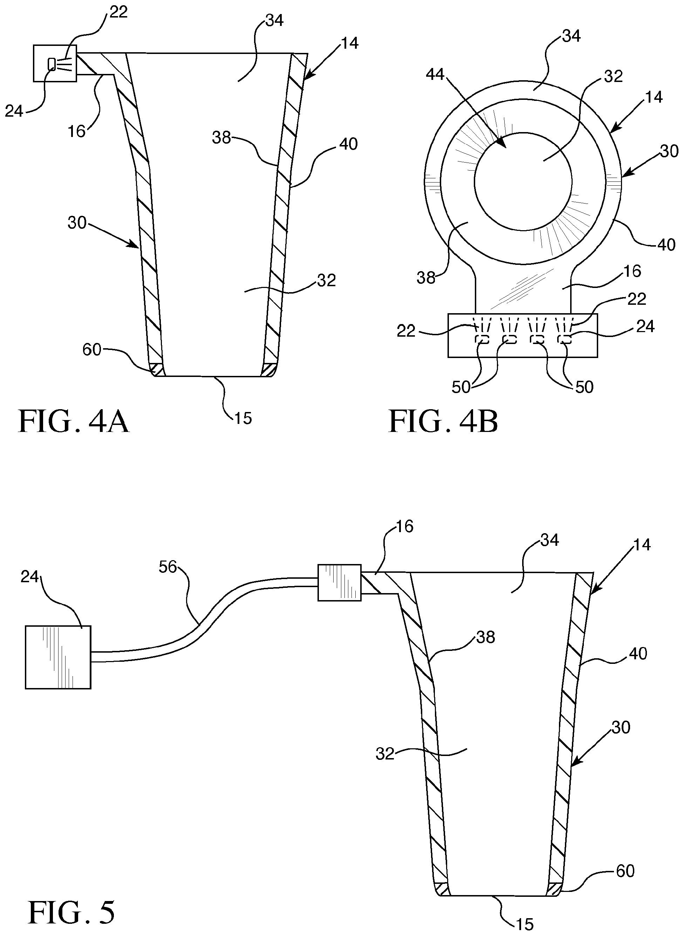

In the embodiment shown in FIGS. 4A and 4B, the surgical cannula 10 includes the light source 24 and the light source is mechanically supported by the tab 18, such that the light emitted 22 by the light source 24 is received by the tab 18. The light 22 received by the tab 18 may be transmitted from the tab 18 to the walls 40 of the walled passageway 30 and then within the walls towards the tip 15 of the walled passageway 30.

In the embodiment shown in FIG. 5, the light source 24 is not supported by the tab 18, but instead the light source 24 is optically connected to the tab 18 via a light guide 56 configured to transfer the light emitted by the light source to the tab 18. For example, the light source 24 may be an external light source optically connected to the tab 18 via an optical fiber.

In the embodiments shown in FIGS. 3A, 3B, 4A, 4B, and 5, the walled passageway 30 acts as a light guide for transporting the received light 22 from the light source towards the open tip 15. The light-extracting features extract the light being transmitted by the walls 40, such that the extracted light illuminates the area 44 bounded by the open tip 15 and/or tissues adjacent the outer surface 40 of the walled passageway 30 as described above.

The light extracting properties of the light-extracting features may vary along the walled passageway 30, such that illumination inside and outside of the working channel 32 appears uniform. For example, illumination of the area 44 bounded by the open tip 15 may vary in brightness by less than 40%, less than 30%, or less than 20%. Similarly, the illumination of the environment adjacent the outer wall 40 may vary in brightness by less than 40%, less than 30%, or less than 20%.

The light-extracting features may be configured to extract at least 60% of the light received by the walled passage 30, such that at most 40% of the received light is emitted from the open tip 15 of the walled passage 30. That is, the light-extracting features are configured to extract most of the light from the walled passageway 30 before the light reaches the open tip 15.

The light-extracting features may be used to control the uniformly of illumination provided by the surgical cannula 10. The light-extracting features may be any suitable structure for extracting light from a structure (such as a light guide) (e.g., to target a specific light output distribution). For example, the light-extracting features may include at least one of surface aberrations, micro-lenses, reflective spots, partial reflective planes, or diffraction gratings. Alternatively or additionally, a diffuser sheet or a 2-D lensing sheet may be (1) placed on an emission surface. In one embodiment, the surface aberrations include at least one of a contour of the surface, surface depositions, or surface etchings.

As shown in FIGS. 3A and 3B, the surgical cannula 10 may include a blocking agent 60 configured to optically attenuate light. For example, the open tip 15 may include the blocking agent 60, such that light emitted from the open tip 15 of the walled passageway 30 is reduced. Similarly, the entrance 34 may include the blocking agent 60, such that light emitted from the entrance 34 of the walled passageway 30 is reduced.

The blocking agent 60 may be any suitable material for attenuating light. For example, as shown in FIG. 3A, the blocking agent 60 may be material added to the walled passageway (such as dark plastic). As another example, the blocking agent 60 may be coloring added or applied to the walled passageway (such as dark paint or dye).

In one embodiment, the surgical cannula 10 also includes a power source 58 electrically connected to the light source 24. In addition to supporting the light source 24, the tab 18 and/or walled passageway 30 may also mechanically support the power source 58. The power source 58 may be any suitable source of electrical energy (e.g., a battery) for supplying electrical power to the light source 24.

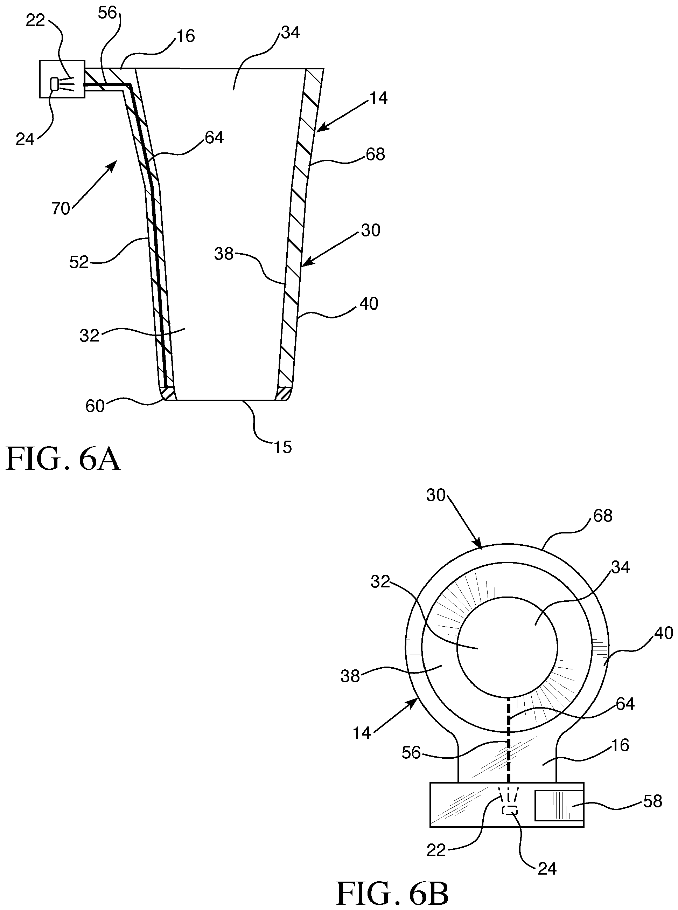

In the embodiment shown in FIGS. 6A and 6B, the surgical cannula 10 includes a light guide 56 and an illuminator 64. The light guide 56 is configured to receive the light 22 emitted by the light source 24 and to transmit the received light via total internal reflection to the illuminator 64. The illuminator 64 is configured to receive the light transmitted by the light guide 56 and to emit the light, such that the light is emitted from the inner surface 38 of the walled passageway 30 into the working channel 32 and illuminates the area 44 bounded by the open tip 15 the walled passageway 30.

In one embodiment, the illuminator 64 is positioned on one side of the walled passageway 30. The illuminator 64 may include light-extracting features for emitting light towards and away from the working channel 32. The light directed away from the working channel 32 may illuminate tissues outside of and on the same side of the walled passageway as the illuminator 64 (i.e., so that these tissues are visible to a surgeon looking through the walled passageway 30). The light directed towards the working channel 32 may be used to illuminate the area 44 bound by the open tip 15. Additionally, the light directed towards the working channel 32 may pass through both the working channel 32 and an opposite portion 68 of the walled passageway 30 located opposite the illuminator 64. In this way, any tissues located on an opposite side of the walled passageway 30 are also illuminated.

Embodiments of the surgical cannula 10 including the illuminator 64 may not include light-extracting features in the walled passageway 30. Instead, the light-extracting features may be present in the illuminator 64. Although, in some embodiments the surgical cannula 10 includes light-extracting features in both the illuminator 64 and the walled passageway 30.

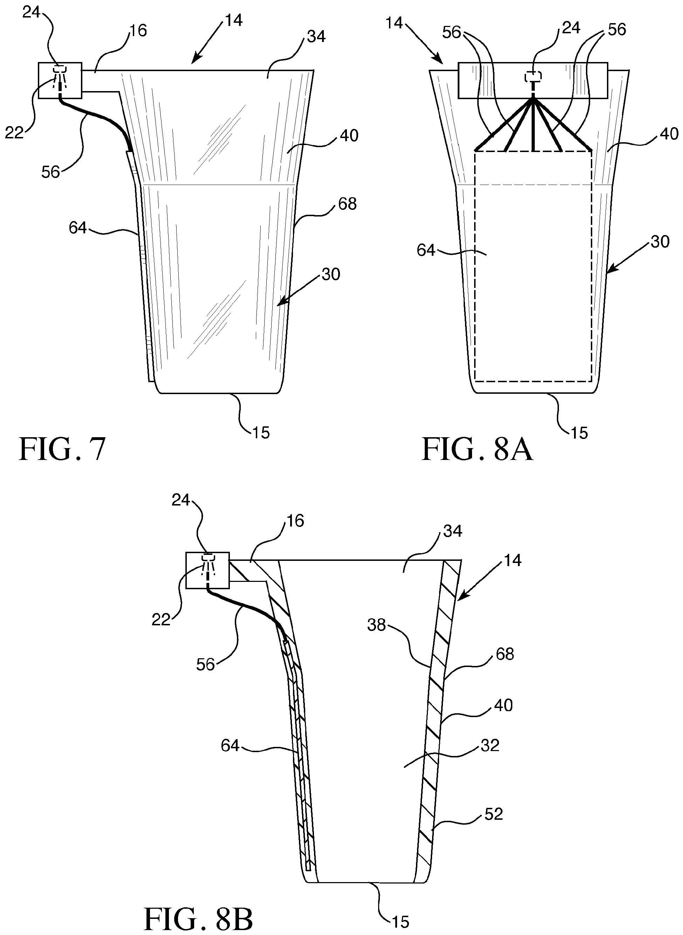

As shown in FIG. 6A, the illuminator 64 may be encapsulated within the walls 52 of the walled passageway 30, such that the illuminator is segregated from an external environment 70. For example, the illuminator 64 may be inserted into a channel in the wall 52 of the walled passageway 30. In the embodiment shown in FIG. 7, the illuminator 64 is attached to the outer surface 40 of the walled passageway 30. For example, the illuminator 64 may be adhered to the outer surface 40. In one embodiment, a coating may also be applied to the illuminator 64, such that the illuminator 64 is separated from and does not come in contact with the external environment 70.

As shown in FIGS. 6A, 6B, and 7, the illuminator 64 may be a linear light guide including light-extracting features configured to extract light from the linear light guide. For example, the illuminator 64 may be a fiber optic (e.g., monofilament fiber optic). Alternatively, as shown in FIGS. 8A and 8B, the illuminator 64 may be a planar light guide including light-extracting features configured to extract light from the planar light guide.

In one embodiment, the light source 24 may be attached to a clip for attaching to the tab 18. The clip may be used to maintain a position of the light source 24 relative to the tab 18 and/or a light guide 56. In another embodiment, the light guide 56 is attached to a clip that is used to optically connect an external light source to the tab 18. For example, the clip may have a circular or semicircular shape that attaches to the entrance 34 of the walled passageway 30.

The light source 24 (and light emitters 50) may be any suitable structure for emitting electromagnetic radiation. For example, the light source 24 may include one or more light emitting diodes (LEDs), organic LEDs (OLEDs), micro-LEDs, laser diodes, mini-LED, quantum dot (QD)-conversion, phosphor conversion, excimer lamps, multi-photon combination, or SLM wavefront manipulation.

The light 22 may include any suitable wavelengths of light. In addition to supplying light to improve visibility of a surgical cavity, the light source 24 may also include light having wavelengths useful for disinfecting the surgical cavity or for performing photobiomodulation. For example, the light 22 may include wavelengths (e.g., 600-1200nm) configured to stimulate wound healing. As an example, the light source 24 may include multiple light emitters. One or more of the light emitters may emit the white light for visibility while other light emitter(s) emit photobiomodulation light.

In one embodiment, the surgical cannula 10 is used to illuminate a cranial surgical cavity or central nervous system surgical procedure. The surgical cannula 10 may be single use or sterilizable (i.e., multi-use).

All ranges and ratio limits disclosed in the specification and claims may be combined in any manner. Unless specifically stated otherwise, references to "a," "an," and/or "the" may include one or more than one, and that reference to an item in the singular may also include the item in the plural.

Although the invention has been shown and described with respect to a certain embodiment or embodiments, equivalent alterations and modifications falling within the scope of the accompanying claims may occur to others skilled in the art upon the reading and understanding of this specification and the annexed drawings. In particular regard to the various functions performed by the above described elements (components, assemblies, devices, compositions, etc.), the terms (including a reference to a "means") used to describe such elements are intended to correspond, unless otherwise indicated, to any element which performs the specified function of the described element (i.e., that is functionally equivalent), even though not structurally equivalent to the disclosed structure which performs the function in the herein illustrated exemplary embodiment or embodiments of the invention. In addition, while a particular feature of the invention may have been described above with respect to only one or more of several illustrated embodiments, such feature may be combined with one or more other features of the other embodiments, as may be desired and advantageous for any given or particular application. The scope of the invention is defined by the claims.

## Claims

1. A surgical cannula (10) for illuminating a surgical cavity using light (22) emitted by a light source (24), the surgical cannula (10) comprising:
an optically transparent walled passageway (30) having an interior working channel (32), an entrance (34), an open tip (15), an inner surface (38), an outer surface (40), and light-extracting features;
whereby the light source (24) comprises multiple light emitters (50) configured to emit the light (22), wherein each of the light emitters (50) is positioned along the entrance of the walled passageway (30), such that the light emitted by each of the light emitters (50) is transported via total internal reflection towards the open tip (15) of the walled passageway (30); and
wherein the light-extracting features are configured to extract the transported light from the walled passageway (30), such that the extracted light is emitted from the inner surface (38) of the walled passageway (30) into the working channel (32) and illuminates an area (44) bounded by the open tip (15) of the walled passageway (30).

2. The surgical cannula (10) of claim 1, wherein the light-extracting features include:
inward extracting structures configured to extract the transported light (22), such that the extracted light illuminates the area bounded (44) by the open tip (15) of the walled passageway (30); and
outward extracting structures configured to extract the transported light (22), such that the extracted light is emitted from the outer surface (40) of the walled passageway (30).

3. The surgical cannula (10) of claim 1 or claim 2, wherein each of the light emitters (50) is enclosed within walls of the walled passageway (30).

4. The surgical cannula (10) of any one of the preceding claims, further comprising a dilator (12) having a closed tip (13), wherein:
the dilator (12) is configured to be received within the interior working channel (32), such that the closed tip (13) of the dilator (12) extends through and past the open tip (15) of the walled passageway (30).

5. The surgical cannula (10) of any one of the preceding claims, wherein light extracting properties of the light-extracting features varies along the walled passageway (30), such that illumination inside and outside of the working channel (32) appears uniform.

6. The surgical cannula (10) of any one of the preceding claims, further comprising a power source (58) mechanically supported by the walled passageway (30).

7. The surgical cannula (10) of any one of the preceding claims, wherein the light-extracting features extract at least 60% of the light (22) received by the walled passage (30), such that at most 40% of the received light is emitted from the open tip (15) of the walled passage (30).

8. The surgical cannula (10) of claim 1 or any one of the preceding claims, wherein at least one of:
the open tip (15) includes a blocking agent (60) configured to optically attenuate the transported light (22), such that light emitted from the open tip (15) of the walled passageway is reduced; or
the entrance (34) includes the blocking agent (60) configured to optically attenuate the light emitted by the light source (24), such that light emitted from the entrance (34) of the walled passageway (30) is reduced.

## Patentansprüche

1. Chirurgische Kanüle (10) zum Beleuchten einer chirurgischen Höhle unter Verwendung von Licht (22), das durch eine Lichtquelle (24) emittiert wird, wobei die chirurgische Kanüle (10) Folgendes umfasst:
einen optisch transparenten mit Wänden versehenen Durchgang (30), der einen inneren Arbeitskanal (32), einen Eingang (34), eine offene Spitze (15), eine Innenfläche (38), eine Außenfläche (40) und Lichtextraktionsmerkmale aufweist;
wodurch die Lichtquelle (24) mehrere Lichtemitter (50) umfasst, die konfiguriert sind, um das Licht (22) zu emittieren, wobei jeder von den Lichtemittern (50) entlang des Eingangs des mit Wänden versehenen Durchgangs (30) positioniert ist, sodass das Licht, das durch jeden der Lichtemitter (50) emittiert wird, über Totalinnenreflexion zu der offenen Spitze (15) des mit Wänden versehenen Durchgangs (30) transportiert wird; und
wobei die Lichtextraktionsmerkmale konfiguriert sind, um das transportierte Licht von dem mit Wänden versehenen Durchgang (30) zu extrahieren, sodass das extrahierte Licht von der Innenfläche (38) des mit Wänden versehenen Durchgangs (30) in den Arbeitskanal (32) emittiert wird und einen Bereich (44) beleuchtet, der durch die offene Spitze (15) des mit Wänden versehenen Durchgangs (30) begrenzt ist.

2. Chirurgische Kanüle (10) nach Anspruch 1, wobei die Lichtextraktionsmerkmale Folgendes beinhalten:
nach innen gerichtete Extraktionsstrukturen, die konfiguriert sind, um das transportierte Licht (22) zu extrahieren, sodass das extrahierte Licht den Bereich beleuchtet, der durch die offene Spitze (15) des mit Wänden versehenen Durchgangs (30) begrenzt ist (44); und
nach außen gerichtete Extraktionsstrukturen, die konfiguriert sind, um das transportierte Licht (22) zu extrahieren, sodass das extrahierte Licht von der Außenfläche (40) des mit Wänden versehenen Durchgangs (30) emittiert wird.

3. Chirurgische Kanüle (10) nach Anspruch 1 oder Anspruch 2, wobei jeder von den Lichtemittern (50) innerhalb von Wänden des mit Wänden versehenen Durchgangs (30) eingeschlossen ist.

4. Chirurgische Kanüle (10) nach einem der vorhergehenden Ansprüche, ferner umfassend einen Dilatator (12) mit einer geschlossenen Spitze (13), wobei:
der Dilatator (12) konfiguriert ist, um innerhalb des inneren Arbeitskanals (32) aufgenommen zu sein, sodass sich die geschlossene Spitze (13) des Dilatators (12) durch die offene Spitze (15) des mit Wänden versehenen Durchgangs (30) hindurch und darüber hinaus erstreckt.

5. Chirurgische Kanüle (10) nach einem der vorhergehenden Ansprüche, wobei Lichtextraktionseigenschaften der Lichtextraktionsmerkmale entlang des mit Wänden versehenen Durchgangs (30) variieren, sodass Beleuchtung innerhalb und außerhalb des Arbeitskanals (32) gleichmäßig erscheint.

6. Chirurgische Kanüle (10) nach einem der vorhergehenden Ansprüche, ferner umfassend eine Leistungsquelle (58), die mechanisch durch den mit Wänden versehenen Durchgang (30) gestützt wird.

7. Chirurgische Kanüle (10) nach einem der vorhergehenden Ansprüche, wobei die Lichtextraktionsmerkmale zumindest 60 % des Lichts (22) extrahieren, das durch den mit Wänden versehenen Durchgang (30) empfangen wird, sodass höchstens 40 % des empfangenen Lichtes von der offenen Spitze (15) des mit Wänden versehenen Durchgangs (30) emittiert werden.

8. Chirurgische Kanüle (10) nach Anspruch 1 oder einem der vorhergehenden Ansprüche, wobei zumindest eines von Folgendem gilt:
die offene Spitze (15) beinhaltet ein Blockiermittel (60), das konfiguriert ist, um das transportierte Licht (22) optisch zu dämpfen, sodass Licht, das von der offenen Spitze (15) des mit Wänden versehenen Durchgangs emittiert wird, reduziert wird; oder
der Eingang (34) beinhaltet das Blockiermittel (60), das konfiguriert ist, um das Licht, das durch die Lichtquelle (24) emittiert wird, optisch zu dämpfen, sodass Licht, das von dem Eingang (34) des mit Wänden versehenen Durchgangs (30) emittiert wird, reduziert wird.

## Revendications

1. Canule chirurgicale (10) destinée à éclairer une cavité chirurgicale à l'aide d'une lumière (22) émise par une source de lumière (24), la canule chirurgicale (10) comprenant :
un passage à parois optiquement transparent (30) comportant un canal de travail intérieur (32), une entrée (34), une pointe ouverte (15), une surface interne (38), une surface externe (40) et des éléments d'extraction de lumière ;
moyennant quoi la source de lumière (24) comprend de multiples émetteurs de lumière (50) conçus pour émettre la lumière (22), chacun des émetteurs de lumière (50) étant positionné le long de l'entrée du passage à parois (30), de sorte que la lumière émise par chacun des émetteurs de lumière (50) soit transportée par réflexion interne totale vers la pointe ouverte (15) du passage à parois (30) ; et
lesdits éléments d'extraction de lumière étant conçus pour extraire la lumière transportée du passage à parois (30), de sorte que la lumière extraite soit émise depuis la surface interne (38) du passage à parois (30) dans le canal de travail (32) et éclaire une zone (44) délimitée par la pointe ouverte (15) du passage à parois (30).

2. Canule chirurgicale (10) selon la revendication 1, lesdits éléments d'extraction de lumière comprenant :
des structures d'extraction vers l'intérieur conçues pour extraire la lumière transportée (22), de sorte que la lumière extraite éclaire la zone délimitée (44) par la pointe ouverte (15) du passage à parois (30) ; et
des structures d'extraction vers l'extérieur conçues pour extraire la lumière transportée (22), de sorte que la lumière extraite soit émise à partir de la surface externe (40) du passage à parois (30).

3. Canule chirurgicale (10) selon la revendication 1 ou la revendication 2, chacun des émetteurs de lumière (50) étant enfermé dans les parois du passage à parois (30).

4. Canule chirurgicale (10) selon l'une quelconque des revendications précédentes, comprenant en outre un dilatateur (12) comportant une pointe fermée (13) :
ledit dilatateur (12) étant conçu pour être reçu à l'intérieur du canal de travail intérieur (32), de sorte que la pointe fermée (13) du dilatateur (12) s'étendent à travers et au-delà de la pointe ouverte (15) du passage à parois (30).

5. Canule chirurgicale (10) selon l'une quelconque des revendications précédentes, lesdites propriétés d'extraction de lumière des éléments d'extraction de lumière variant le long du passage à parois (30), de sorte que l'éclairage à l'intérieur et à l'extérieur du canal de travail (32) semble uniforme.

6. Canule chirurgicale (10) selon l'une quelconque des revendications précédentes, comprenant en outre une source d'alimentation (58) supportée mécaniquement par le passage à parois (30).

7. Canule chirurgicale (10) selon l'une quelconque des revendications précédentes, lesdits éléments d'extraction de lumière extrayant au moins 60 % de la lumière (22) reçue par le passage à parois (30), de sorte qu'au plus 40 % de la lumière reçue soit émise par la pointe ouverte (15) du passage à parois (30).

8. Canule chirurgicale (10) selon la revendication 1 ou l'une quelconque des revendications précédentes, au moins l'un des éléments suivants :
ladite pointe ouverte (15) comprenant un agent de blocage (60) conçu pour atténuer optiquement la lumière transportée (22), de sorte que la lumière émise par la pointe ouverte (15) du passage à parois soit réduite ; ou
ladite entrée (34) comprenant l'agent de blocage (60) conçu pour atténuer optiquement la lumière émise par la source de lumière (24), de sorte que la lumière émise par l'entrée (34) du passage à parois (30) soit réduite.
